# EUROPEAN PATENT SPECIFICATION

(11) **EP 3 484 427 B1**
(45) Date of publication and mention of the grant of the patent: **14.04.2021**
(21) Application number: 17742907.3
(22) Date of filing: 11.07.2017
(51) Int. Cl.: A61F 13/49, A61F 13/494, A61F 13/496

(54) **PROTECTIVE GARMENT FOR PREVENTING BODILY FLUID LEAKAGE**
SCHUTZKLEIDUNG ZUM VERHINDERN DES AUSTRITTS VON KÖRPERFLÜSSIGKEITEN
VÊTEMENT DE PROTECTION DESTINÉ À PRÉVENIR LES FUITES DE FLUIDE CORPOREL

(30) Priority: 12.07.2016 US 201662361216 P
(43) Date of publication of application: 22.05.2019
(73) Proprietor: The LYCRA Company UK Limited, Manchester M2 3DE (GB)
(72) Inventor: BISSAH, Kofi, Newark, Delaware 19702 (US)
(74) Representative: Cockerton, Bruce Roger
(86) International application number: PCT/US2017/041528
(87) International publication number: WO 2018/013561

(56) References cited:
- WO-A1-99/25289
- WO-A1-99/25298
- WO-A1-2014/176677
- US-A- 5 722 127
- US-A1- 2014 018 757

## Description

### Field of the Invention

The present invention relates to garments, and more particularly, to protective garments constructed to effectively prevent bodily fluid leakage.

### Background of the Invention

Various protective undergarments currently available in the market are designed and constructed to prevent bodily fluid from leaking onto wearer's inner and outer apparel. Generally, feminine sanitary products such as sanitary napkins, pads and tampons are integrated with these undergarments to absorb very large amounts of bodily fluid generated from menstruation and/or adult incontinence. However, these protective undergarments have some drawbacks. First, they are not designed and made to provide adequate support to prevent the sanitary products from sagging caused by the heavy weight of the absorbed bodily fluid. Second, designs and fabrics of the protective undergarments also do not provide adequate protection against bodily fluid from leaking onto inner and outer apparel. Finally, many protective undergarments in the market are manufactured with fabrics that are uncomfortable and aesthetically unpleasing since these undergarments need to compliment the thick feminine sanitary products for absorbing large volume of bodily fluid For example, US2014/0018757 discloses a containment system with a chassis, a sling, and a fluid-impervious pouch, wherein the sling is minimally attached to the chassis to maximise the fit and natural movement of the chassis.

Thus, while these protective garments offer some protection, these are not suitable for an undergarment for daily use.

Accordingly, although various garments for providing bodily fluid leakage protection are available currently in the market, there is a need for fashionable protective garments constructed to effectively prevent bodily fluid leakage.

### Summary of the Invention

According to the present invention, there is provided a garment for preventing bodily fluid leakage and sagging of an absorbent pad includes an undergarment and a sling placed adjacent to a crotch portion of the undergarment, the sling designed and configured to provide a longitudinal stretch to move with a body of a wearer and to exert a garment force against the absorbent pad and the body, and wherein the sling is removable.

This and other aspects of the present invention will be better understood in view of the following detailed description.

### Detailed Description of Preferred Embodiments

The present invention is directed to a protective garment that provides improved absorption and bodily fluid leakage protection for a woman's menstruation or adult incontinence. In addition, the protective garment according to an embodiment of the present invention is constructed with an attractive look and feel, similar to fashionable underwear, and provides comfort while performing daily activities. The aforementioned characteristics of the protective garment are achieved by the use of a unique fabric material and design, as will be described in greater detail below. The protective garment includes an undergarment, a sling and an absorbent pad which is dimensioned to fit within the sling. A combination of the sling and the absorbent pad provides light and comfortable compression that enhances bodily fluid leakage protection. Specifically, compressive forces generated from the sling and the absorbent pad allows the absorbent pad to absorb and contain high volume of bodily discharges from a woman's menstrual cycle and/or adult incontinence, thereby preventing bodily fluid leakage and sagging of the absorbent pad.

The undergarment includes a body portion and a crotch portion which is integrally connected to the body portion to form the bottom of the undergarment. The undergarment defines leg openings that are formed by the bottom region of the body portion and the sides of the crotch portion. Elastic encircling bands could be provided around the leg openings so that they could fit snugly around wearer's thighs and provide additional protection against bodily fluid leakage. The body portion of the undergarment is made of one or more materials having suitable properties for a desired application, including strength, weight, rigidity, etc.

The sling is a woven or nonwoven material which is formed to a desired shape to fixedly hold a sanitary pad or napkin in place proximate to the crotch portion of a panty or garment. The sling is placed adjacent to the crotch portion of the undergarment such that it provides a longitudinal stretch to move with a body of the wearer. The sling, as will be described in greater detail below, is constructed with a material that is suitable to exert a garment force against the absorbent pad and the body. The garment force is a light and comfortable compressive force that pushes the absorbent pad against the body of the wearer, thereby immobilizing the absorbent pad while the wearer performs daily activities. This immobilization of the absorbent pad maximizes its absorption of bodily fluid from the wearer, thereby enhancing bodily fluid leakage protection. In addition, the garment force provides strong compression against the absorbent pad to prevent sagging of the absorbent pad caused by the heavy weight of the absorbed bodily fluid. The sling designed to be removable from the undergarment. The removable sling can be attached and unattached using any suitable fastening means (snaps, buttons, hook and loop fasteners, adhesive, zippers, and the like). Additionally, the sling can be formed with the pad as a single unit; and may be disposable or reusable.

The shape of the sling can be any shape provided that it has enough coverage to hold the napkin in place without shifting. Examples include but are not limited to an hourglass or undulating shaped sling extending from front to back and having a front lobe, a back lobe and a crotch section with a narrowing through the crotch forming the inner part or sling; an hour glass shaped sling with back and front lobes each having wider widths than a crotch section; and a trapezoidal shaped sling with a narrow width at the back lobe and wider width at the crotch and front lobe.

The absorbent pad is shaped and dimensioned to fit within the sling such that the sling compliments the absorbent pad during the woman's menstruation and/or adult incontinence. The absorbent pad exerts a pad force against the body of the wearer. A combination of the garment force exerted from the sling and the pad force exerted from the absorbent pad minimizes radial distribution of bodily fluid and promotes longitudinal fluid transport, thus providing enhanced bodily fluid containment and minimizing bodily fluid leakage. The sling may be constructed of one piece or multiple segments formed in a manner to hold the absorbent pad in place. When the sling is formed of multiple segments the segments may be of the same materials or differing materials. Sling constructs may comprise a single material piece; an elastomeric member that imparts stretch characteristics; interwoven support strands or cords, adhesive supports; two or more portions comprising a separate front and back attachment means to hold a pad in place, or various other configurations suited to hold the absorbent pad in place.

The sling is made out of a material that contains elastic composite structures. Advantageously, these structures provide a high compressive force against the absorbent pad and the body for maximizing absorbent pad utilization, resulting enhanced bodily fluid containment. In addition, the sling material makes the protective garment comfortable and aesthetically pleasing to wear on a daily basis.

The sling material includes a substrate, a polyurethane material, and an adhesive. For purposes of this invention, the term "substrate" is used in its broadest sense to mean a flexible or deformable structure or element which has at least one surface onto which the selected elasticizing polyurethane materials used herein can be adhesively bonded. Such substrates will generally be flexible substrates with two surfaces, e.g., upper and lower, and can be in the form of films, woven or knitted substrates, mesh or scrim substrates or nonwoven substrates. Such substrates will also be relatively inelastic. Relatively inelastic substrates are those which can be elongated no more than about 120% in any direction without rupture or those which exhibit growth of more than 30% of the elongated length after elongation to 50% of the break elongation and removal of the elongating force.

Preferred relatively inelastic substrates for elasticizing herein will be in the form of nonwoven substrates. Nonwoven substrates or "webs" are substrates having a structure of individual fibers, filaments or threads that are interlaid, but not in an identifiable, repeating manner. Nonwoven substrates can be formed by a variety of conventional processes such as, for example, meltblowing processes, spunbonding processes and bonded carded web processes.

Meltblown substrates or webs are those made from meltblown fibers. Meltblown fibers are formed by extruding a molten thermoplastic material through a plurality of fine, usually circular, die capillaries as molten thermoplastic material or filaments into a high velocity gas (e.g. air) stream. This attenuates the filaments of molten thermoplastic material to reduce their diameter, which may be to microfiber diameter. Thereafter, the meltblown fibers are carried by the high velocity gas stream and are deposited on a collecting surface to form a web of randomly disbursed meltblown fibers. Such a process is disclosed, for example, U.S. Patent No. 3, 849, 241.

Spunbonded substrates or "webs" are those made from spunbonded fibers. Spunbonded fibers are small diameter fibers formed by extruding a molten thermoplastic material as filaments from a plurality of fine, usually circular, capillaries of a spinerette. The diameters of the extruded filaments are then rapidly reduced as by, for example, eductive stretching or other well-known spun-bonding mechanisms. The production of spun-bonded nonwoven webs is illustrated, for example, in U.S. Patent Nos. 3,692,618 and 4, 340, 563.

The relatively inelastic substrates can be constructed from a wide variety of materials. Suitable materials, for example, can include: polyethylene, polypropylene, polyesters such as polyethylene terephthalate, polybutane, polymethyidentene, ethylenepropylene co-polymers, polyamides, tetrablock polymers, styrenic block copolymers, polyhexamethylene adipamide, poly-(oc-caproamide), polyhexamethylenesebacamide, polyvinyls, polystyrene, polyurethanes, polytrifluorochloroethylene, ethylene vinyl acetate polymers, polyetheresters, cotton, rayon, hemp and nylon. In addition, combinations of such material types may be employed to form the relatively inelastic substrates to be elasticized herein.

Preferred substrates to be elasticized herein include structures such as polymeric spunbonded nonwoven webs. Particularly preferred are spunbonded polyolefin nonwoven webs having a basis weight of from about 10 to about 40 grams/m². More preferably such structures are polypropylene spunbonded nonwoven webs having a basis weight of from about 14 to about 25 grams/m². A particular type of preferred composite structure in the form of a multilayer laminate using nonwovens of this type is described in greater detail hereinafter.

The relatively inelastic substrates as hereinbefore described can be elasticized by adhesively bonding to one or more of such substrates a certain type of elastomeric polyurethane material. Such adhesive bonding to the substrate to be elasticized occurs while the polyurethane material is drafted to an elongated state.

The spandex fiber of the present invention meets the definition of "a manufactured fiber in which the fiber-forming substance is a long chain synthetic polymer comprised of at least 85% of a segmented polyurethane". The elastic properties and the retention of the elastic properties after heat treatment of a spandex fiber are very much dependent on the content of the segment polyurethane, and the chemical composition, the micro domain structure and the polymer molecular weight of the segment polyurethane. As it has been well established, segmented polyurethanes are one family of long chain polyurethanes consisting of hard and soft segments by step polymerization of a hydroxyl-terminated polymeric glycol, a diisocyanate and a low molecular weight chain extender. Depending on the nature of the chain extender used, a diol or a diamine, the hard segment in the segmented polyurethane can be urethane or urea. The segmented polyurethanes with urea hard segments are categorized as polyurethaneureas. In general, the urea hard segment forms stronger inter-chain hydrogen bondings functioning as physical cross-link points, than the urethane hard segment. Therefore, a diamine chain extended polyurethaneurea typically has better formed crystalline hard segment domains with higher melting temperatures and better phase separation between soft segments and hard segments than a short chain diol extended polyurethane. Because of the integrity and resistivity of the urea hard segment to thermal treatment, polyurethaneurea are typically spun into fibers through a solution spinning process, either wet spinning or dry spinning. Polyurethane fibers, produced with urethane hard segments, and selected polyurethaneurea fibers may also be produced by melt spinning.

A mixture or blend of two or more segmented polyurethanes or polyurethaneureas can be used. Optionally, a mixture or blend of the segmented polyurethaneurea can also be used with another segmented polyurethane or other fiber forming polymers.

The polyurethane or polyurethaneurea is made by a two-step process. In the first step, an isocyanate-terminated urethane prepolymer is formed by reacting a polymeric glycol with a diisocyanate. Typically, the molar ratio of the diisocyanate to the glycol is controlled in a range of 1.50 to 2.50. If desired, catalyst can be used to assist the reaction in this prepolymerization step. In the second step, the urethane prepolymer is dissolved in a solvent such as N,N-dimethylacetamide (DMAc) and is chain extended with a short chain diamine or a mixture of diamines to form the polyurethaneurea solution. The polymer molecular weight of the polyurethanurea is controlled by small amount of mono-functional alcohol or amine, typically less than 60 miliequivalent per kilogram of the polyurethaneurea solids, added and reacted in the first step and/or in the second step. The additives can be mixed into the polymer solution at any stage after the polyurethaneurea is formed but before the solution is spun into the fiber. The total additive amount in the fiber is typically less than 10% by weight. The solid content including the additives in the polymer solution prior to spinning is typically controlled in a range of 30.0% to 40.0% by weight of the solution. The solution viscosity is typically controlled in range from 2000 to 5000 poises for optimum spinning performance. Suitable segmented polyurethane polymers can also be made in the melt, provided that the hard segment melting point is low enough.

Suitable polymeric glycols for the polyurethaneurea include polyether glycols, polycarbonate glycols, and polyester glycols of number average molecular weight of about 600 to about 3,500. Mixtures of two or more polymeric glycol or copolymers can be included.

Examples of polyether glycols that can be used include those glycols with two terminal hydroxy groups, from ring-opening polymerization and/or copolymerization of ethylene oxide, propylene oxide, trimethylene oxide, tetrahydrofuran, and 3-methyltetrahydrofuran, or from condensation.

Polymerization of a polyhydrolic alcohol, such as a diol or diol mixtures, with less than 12 carbon atoms in each molecule, such as ethylene glycol, 1,3-propanediol, 1,4-butanediol, 1,5-pentanediol 1,6-hexanediol, 2,2-dimethyl-1,3 propanediol, 3-methyl-1,5-pentanediol, 1,7-heptanediol, 1,8-octanediol, 1,9-nonanediol, 1,10-decanediol and 1,12-dodecanediol. A linear, bifunctional polyether polyol is preferred, and a poly(tetramethylene ether) glycol with umber average molecular weight of about 1,700 to about 2,100, such as Terathane® 1800 (INVISTA of Wichita, Kans.) with a functionality of 2, is one example of the specific suitable glycols. Co-polymers can include poly(tetramethylene ether co-ethylene ether) glycol and poly(2-methyl tetramethylene ether co- tetramethylene ether) glycol.

Examples of polyester glycols that can be used include those ester glycols with two terminal hydroxy groups, produced by condensation polymerization of aliphatic polycarboxylic acids and polyols, or their mixtures, of low molecular weights with no more than 12 carbon atoms in each molecule. Examples of suitable polycarboxylic acids are malonic acid, succinic acid, glutaric acid, adipic acid, pimelic acid, suberic acid, azelaic acid, sebacic acid, undecanedicarboxylic acid, and dodecanedicarboxylic acid. Examples of suitable glycols for preparing the polyester polyols are ethylene glycol, 1,3-propanediol, 1,4-butanediol, 1,5-pentanediol 1,6-hexanediol, neopentyl glycol, 3-methyl-1,5- pentanediol, 1,7-heptanediol, 1,8-octanediol, 1,9-nonanediol, 1,10-decanediol and 1,12 dodecanediol. A linear bifunctional polyester polyol with a melting temperature of about 5°C to about 50°C is an example of a specific polyester glycol.

Examples of polycarbonate glycols that can be used include those carbonate glycols with two terminal hydroxyl groups, produced by condensation polymerization of phosgene, chloroformic acid ester, dialkyl carbonate or diallyl carbonate and aliphatic polyols, or their mixtures, of low molecular weights with no more than 12 carbon atoms in each molecule. Examples of suitable polyols for preparing the polycarbonate polyols are diethylene glycol, 1,3-propanediol, 1,4-butanediol, 1,5-pentanediol, 1,6-hexanediol, neopentyl glycol, 3-metl-iyl-1,5-pentanediol, 1,7-heptanediol, 1,8-octanediol, 1,9-nonanediol, 1,10-decanediol and 1,12-dodecanediol. A linear, bifunctional polycarbonate polyol with a melting temperature ofabout 5°C to about 50°C is an example of a specific polycarbonate polyol.

The diisocyanate component used to make the polyurethaneurea can include a single diisocyanate or a mixture of different diisocyanates including an isomer mixture of diphenylmethane diisocyanate (MDI) containing 4,4'-methylene bis(phenyl isocyanate) and 2,4'-methylene bis(phenyl isocyanate). Any suitable aromatic or aliphatic diisocyanate can be included. Examples of diisocyanates that can be used include, but are not limited to 4,4'-methylene bis(phenyl isocyanate), 4,4'-methylenebis(cyclohexyl isocyanate), 1,4-xylenediisocyanate, 2,6-toluenediisocyanate, 2,4-toluenediisocyanate, and mixtures thereof. Examples of specific polyisocyanate components include Takenate® 500 (Mitsui Chemicals), Mondur® MB (Bayer), Lupranate® M (BASF), and Isonate® 125 MDR (Dow Chemical), and combinations thereof.

Examples of suitable diamine chain extenders for making the polyurethaneurea include: 1,2-ethylenediamine; 1,4-butanediamine; 1,2-butanediamine; 1,3-butanediamine; 1,3-diamino-2,2-dimethylbutane; 1,6-hexamethylenediamine; 1,12-dodecanediamine; 1,2-propanediamine; 1,3-propanediamine; 2-methyl-1,5 -pentanediamine; 1-amino-3,3,5-trimethyl-5-aminomethylcyclohexane; 2,4-diamino- 1 -methylcyclohexane; N-methylamino-bis(3-propylamine); 1,2-cyclohexanediamine; 1,4-cyclohexanediamine; 4,4'-methylene-bis (cyclohexylamine); isophorone diamine; 2,2-dimethyl-1,3-propanediamine; meta-tetramethylxylenediamine; 1,3-diamino-4-methylcyclohexane; 1,3-cyclohexane-diamine; 1,1 -methylene-bis(4,4'-diaminohexane); 3-aminomethyl-3,5,5-trimethylcyclohexane; 1,3-pentanediamine(1,3-diaminopentane); m-xylylene diamine; and .Teffamine® (Texaco). Optionally, water and tertiary alcohols such as tert-butyl alcohol and α-Cumyl alcohol can also be used as chain extenders to make the polyurethaneurea.

When a polyurethane is desired, a chain extender or mixture of chain extenders used should be a diol. Examples of such diols that may be used include, but are not limited to, ethylene glycol, 1,3-propanediol, 1,2-propylene glycol, 3-methyl-1,5-pentanediol, 2,2-dimethyl-1,3-trimethylene diol, 2,2,4-trimethyl-1,5-pentanediol, 2-methyl-2-ethyl-1,3-propanediol, 1,4-bis(hydroxyethoxy)benzene, 1,4-butanediol, and mixtures thereof.

A monofunctional alcohol or a primary/secondary monofunctional amine can be included as a chain terminator to control the molecular weight of the polyurethaneurea. Blends of one or moremonofunctional alcohols with one or more monofunctional amines may also be included.

Examples of monofunctional alcohols useful as a chain terminator with the present invention include at least one member selected from the group consisting of aliphatic and cycloaliphatic primary and secondary alcohols with 1 to 18 carbons, phenol, substituted phenols, ethoxylated alkyl phenols and ethoxylated fatty alcohols with molecular weight less than about 750, including molecular weight less than 500, hydroxyamines, hydroxymethyl and hydroxyethyl substituted tertiary amines, hydxoxymethyl and hydroxyethyl substituted heterocyclic compounds, and combinations thereof, including furfuryl alcohol, tetrahydrofurfuryl alcohol, N-(2-hydroxyethyl)succinimide, 4 -(2-hydroxyethyl)morpholine, methanol, ethanol, butanol, neopentyl alcohol, hexanol, cyclohexanol, cyclohexanemethanol, benzyl alcohol, octanol, octadecanol, N,N-diethylhydxoxylamine, 2-(diethylamino) ethanol, 2-dimethylaminoethanol, and 4-piperidineethanol, and combinations thereof. Preferably, such a monofunctional alcohol is reacted in the step of making the urethane prepolymer to control the polymer molecular weight of polyurethaneurea formed at a later step.

Examples of suitable monofunctional primary amines useful as a chain terminator for the polyurethaneurea include, but are limited to, ethylamine, propylamine, isopropylamine, n-butylamine, sec-butylamine, tert-butylamine, isopentylamine, hexylamine, octylamine, ethylhextylamine, tridecylamine, cyclohexylamine, oleylamine and stearylamine. Examples of suitable monofunctional dialkylamine chain blocking agents include: N,N-diethylamine, N-ethyl-N-propylamine, N,N-diisopropylamine, N-tert-butyl-N-methylamine, N-tert-butyl-N-benzylamine, N,N-dicyclohexylamine, N-ethyl-N-isopropylamine, N-tertbutyl-N-isopropylamine, N-isopropyl-N-cyclohexylamine, N-ethyl-N-cyclohexylamine, N,N-diethanolamine, and 2,2,6,6-tetramethylpiperidine. Preferably, such a monofunctional amine is used during the chain extension step to control the polymer molecular weight of the polyurethaneurea. Optionally, amino-alcohols such as ethanolamine, 3-amino-1-propanol, isopropanolamine and N-methylethanolamine can also be used to regulate the polymer molecular weight during the chain extension reaction.

Classes of additives that may be optionally included in spandex fiber are listed below. An exemplary and non-limiting list is included. However, additional additives are well-known in the art. Examples include: antioxidants, UV stabilizers, colorants, pigments, cross-linking agents, phase change materials (paraffin wax), antimicrobials, minerals (i.e., copper), microencapsulated additives (i.e., aloe vera, vitamin E gel, aloe vera, sea kelp, nicotine, caffeine, scents or aromas), nanoparticles (i.e., silica or carbon), calcium carbonate, flame retardants, antitack additives, chlorine degradation resistant additives, vitamins, medicines, fragrances, electrically conductive additives, dyeability and/or dye-assist agents (such as quaternary ammonium salts).

Other additives which may be added to the include adhesion promoters and fusibility improvement additives, anti-static agents, anti-creep agents, optical brighteners, coalescing agents, electroconductive additives, luminescent additives, lubricants, organic and inorganic fillers, preservatives, texturizing agents, thermochromic additives, insect repellants, and wetting agents, stabilizers (hindered phenols, zinc oxide, hindered amine), slip agents (silicone oil) and combinations thereof.

The additive may provide one or more beneficial properties including: dyeability, hydrophobicity (i.e., polytetrafluoroethylene (PTFE)), hydxophilicity (i.e., cellulose), friction control, chlorine resistance, degradation resistance (i.e., antioxidants), adhesiveness and/or fusibility (i.e., adhesives and adhesion promoters), flame retardance, antimicrobial behavior (silver, copper, ammonium salt), barrier, electrical conductivity (carbon black), tensile properties, color, luminescence, recyclability, biodegradability, fragrance, tack control (i.e., metal stearates), tactile properties, set-ability, thermal regulation (i.e., phase change materials), nutriceutical, delustrant such as titanium dioxide, stabilizers such as hydrotalcite, a mixture of huntite and hydromagnesite, UV screeners, and combinations thereof.

Additives may be included in any amount suitable to achieve the desired effect.

The selected types of polyurethanes and polyurethaneureas as hereinbefore described can be used to provide elastomeric material in a wide variety of forms. Solutions of these polyurethane materials can be cast into films or spun into spandex fibers or filaments. Spandex in the form of fibers or filaments is most commonly used in elasticizing substrates.

Spandex fibers can be formed from the polyurethane or polyurethaneurea polymer solution through fiber spinning processes such as dry spinning, wet spinning, or melt spinning. In dry spinning, a polymer solution comprising a polymer and solvent is metered through spinneret orifices into a spin chamber to form a filament or filaments. Polyurethaneureas are typically dry-spun or wet-spun when spandex fibers made therefrom are desired. Polyurethanes are typically melt-spun when spandex fibers made therefrom are desired.

Typically, a polyurethaneurea polymer is dry spun into filaments from the same solvent as has been used for the polymerization reaction. Gas is passed through the chamber to evaporate the solvent to solidify the filament(s), Filaments are dry spun at a windup speed of at least 200 meters per minute. The spandex can be spun at a speed at any desired speed such as in excess of 800 meters/minute. As used herein, the term "spinning speed" refers to the yarn take-up speed.

Good spinability of spandex filaments is characterized by infrequent filament breaks in the spinning cell and in the wind up. The spandex can be spun as single filaments or can be coalesced by conventional techniques into multi-filament yarns. Each filament in multifilament yarn can typically be of textile decitex (dtex), e.g., in the range of 6 to 25 dtex per filament.

Spandex in the form of a single filament or a multifilament yarn is typically used for elasticizing substrates to form the composite structures herein. Multifilament spandex yarn frequently will comprise from about 4 to about 120 filaments per strand of yarn. Spandex filaments or yarns which are especially suitable are those ranging from about 200 to about 3600 decitex, including from about 200 decitex to about 2400 decitex and from about 540 to about 1880 decitex.

The selected type of polyurethane elasticizing agent as hereinbefore described will be in the form of a structure or element which is adhesively bonded or attached to the relatively inelastic substrates being elasticized. Adhesive bonding of the selected type of polyurethane herein to such inelastic flexible substrates is generally brought about through the use of a conventional hot melt adhesive.

Conventional hot melt adhesives are typically thermoplastic polymers which exhibit high initial tack, provide good bond strength between the components and have good ultraviolet and thermal stability. Preferred hot melt adhesives will be pressure sensitive. Examples of suitable hot melt adhesives are those comprising a polymer selected from the group consisting of styrene-isoprene-styrene (SIS) copolymers; styrene-butadiene-styrene (SBS) copolymers; styrene-ethylene-butylene-styrene (SEBS) copolymers; ethylene-vinyl acetate (EVA) copolymers; amorphous poly-alpha-olefin (APAO) polymers and copolymers; and ethylene-styrene interpolymers (ESI). Most preferred are adhesives based on styrene-isoprene-styrene (SIS) block copolymers. Hot melt adhesives are commercially available. They are marketed under designations such as H-2104, H-2494, H-4232 and H-20043 from Bostik; HL-1486 and HL-1470 from H.B. Fuller Company; and NS-34-3260, NS-34-3322 and NS-34-560 from National Starch Company.

### Compressive Force Testing

Four types of undergarments were tested to measure compressive forces exerted against the body of the wearer: undergarment only, undergarment with sling (garment force), undergarment with absorbent pad (pad force), and undergarment with sling and absorbent pad (garment and pad forces). A sensor having four quadrants was used to read and accumulate over 500 data points (pound per square inch (PSI)) during a specific time period (10 seconds). Based on these data points, Table 1 summarizes average PSIs (minimum PSI, mean PSI, maximum PSI) for each quadrant (quadrant 1-4) for each undergarment and sums of PSIs of four quadrants for each undergarment. Each sum represents a total PSI exerted on the body of the wearer. Table 1 is a table summarizing results of compressive forces exerted against a body of a wearer for various undergarments; and Table 1 summarizes results of fluid management efficiency.

**TABLE 1**

| | Geneva w sling | | | Geneva wo sling | | | Geneva w sling+napkin | | | Geneva wo sling+napkin | | |
|---|---|---|---|---|---|---|---|---|---|---|---|---|
| Minimum | | | Maximum | Minimum | | Maximum | Minimum | | Maximum | Minimum | | Maximum |
| Crotch zone | PSI | Mean PSI | PSI | PSI | Mean PSI | PSI | PSI | Mean PSI | PSI | PSI | Moan PSI | PSI |
| quad 1 | 0.023 | 0,025493 | 0.028 | -0.002 | -0.00028 | 0.001 | .0.001 | 0.00046 | 0 | -0.007 | -0.03564 | -0.003 |
| quad 2 | 0.173 | 0.1742 | 0.176 | -0.004 | -0.00276 | -0.001 | 0.052 | 0.055112 | 0.059 | -0.007 | -0.00324 | 0.001 |
| quad 3 | 0.043 | 0.0441 | 0.046 | 0.001 | 0.00236 | 0.005 | 0.007 | 0.006307 | 0.01 | 0 | 0.001673 | 0.004 |
| quad 4 | 0.043 | 0.0441 | 0.046 | 0.011 | 0.012319 | 0.014 | 0.505 | 0.5667 | 0.589 | 0.178 | 0.1099 | 0.305 |
| PSI on body | 0.282 | 0.287893 | 0.296 | 0.006 | 0.012139 | 0.019 | 0.643 | 0.649967 | 0.656 | 0.164 | 0.192693 | 0.307 |

As can be seen from Table 1, the results of the test show that the undergarment with the sling and absorbent pad exerts the highest compressive force range (0.64-0.66 PSI) against the body of the wearer, thereby providing maximum bodily fluid leakage protection.

### Technical Tests

The protective garments (panty nos. 8 and 9 in Table 2), according to the embodiment of the present invention, were tested against the protective garments (panty nos. 1-7 in Table 2) that are currently available in the marketplace. Table 2 is a listing of undergarments or panties that were tested for bodily fluid absorbency and leakage. Specifically, all the panties listed in Table 2 were tested for bodily fluid absorbency and leakage amounts for sideway (sleeping, seating, etc.) and upright (standing) positions using mannequins.

**TABLE 2 - Panties Tested**

| | |
|---|---|
| 1 | Jockey (100% cotton) |
| 2 | Dear Kate (76% Nylont24% Lycra) |
| 3 | Knix by knixweer (65% Nylon/35% Spandex) |
| 4 | Soma (92% Polyamida/8% Spandex) |
| 5 | BALI (100% Nylon) |
| 6 | Ven lty Fair (95% Nylon/5% Spandex) |
| 7 | Bali Santoni Firm Control (80% Nylon/20% Spandex) |
| 8 | Present Invention (Undelachable) |
| 9 | Present Invention (undetachable) |

Table 3 is a table summarizing results of fit characterization and rating of the panties in Table 2;

**TABLE 3 - Fit Characterisation and Rating**

| | | |
|---|---|---|
| 1 | Moderately snug Fit; loose In spots | -/0 |
| 2 | Moderately Tight Fit | 0/+ |
| 3 | Light snug Fit | -/0 |
| 4 | Moderate snug Fit | 0 |
| 5 | Moderately snug Fit | 0 |
| 6 | Moderately snug Fit | 0 |
| 7 | Extremely Tight Fit | + |
| 8 | Extremely Tight Fit | + |
| 9 | Extremely Tight Fit | + |

Table 4 is a table summarizing results of bodily fluid absorbency and leakage for sideway positions of the panties in Table 2;

In addition, the present invention posted the longer wear time before failure than the other panties, as shown in the below Table 4:

**TABLE 4 - Bodily Fluid Absorbency and Leakage (Sideway)**

| | | | |
|---|---|---|---|
| 1 | 5.24 | 36.63 | 2.04 |
| 2 | 11.24 | 76.74 | 2.59 |
| 3 | 6.28 | 43.99 | 0.87 |
| 4 | 6.73 | 47.12 | 0.37 |
| 5 | 3.73 | 20.14 | 1.28 |
| 6 | 6.77 | 47.73 | 2.07 |
| 7 | 7.53 | 52.73 | 1.10 |
| 8 | 17.57 √ | 123.03 | 3.35 |
| 9 | 16.05 √ | 112.40 | 2.99 |

Table 5 is a table summarizing results of bodily fluid absorbency and leakage for upright positions of the panties in Table 2. Table 6 is a table summarizing results of bodily fluid absorbency and leakage at 175 ml insult volume for 25 seconds for upright positions of the panties in Table 2;

**TABLE 6 - Bodily Fluid Absorbency and Leakage (Upright)**

| | | | |
|---|---|---|---|
| 1 | 5.24 | 36.63 | 2.04 |
| 2 | 11,24 | 78.74 | 2.66 |
| 3 | 6.28 | 43.99 | 0.87 |
| 4 | 6.73 | 47.12 | 0,37 |
| 5 | 3.73 | 26.14 | 1.28 |
| 6 | 6.77 | 47.73 | 2.07 |
| 7 | 7.53 | 52.73 | 1.10 |
| B | 17.67 √ | 123.03 | 3.35 |
| 9 | 16.05 √ | 112.40 | 2.99 |

**TABLE 6 - Bodlly Fluid Absorbency and Leakage at 175 ml insult volume for 25 seconds (Sicteway)**

| | | | |
|---|---|---|---|
| 1 | 25 | 175 | 45.59 |
| 2 | 25 | 176 | 45.22 |
| 3 | 25 | 175 | 49.95 |
| 4 | 25 | 175 | 20.76 |
| 6 | 26 | 175 | 51.13 |
| 6 | 25 | 175 | 57.76 |
| 7 | 25 | 175 | 49.56 |
| 8 | 25 | 175 | 26.83 |
| 9 | 26 | 175 | 18.19 |

As can be seen from Tables 2-7, the results of these technical tests show that the present invention performed significantly better than the rest of the panties on both bodily fluid absorbency and leakage protection. In particular, the present invention demonstrated the best bodily fluid efficiency defined by absorption amount (ml) divided by undergarment weight (g), as shown in the Table 7 below:

**TABLE 7- Fluid Management Efficiency (Sideway)**

| | | | | | |
|---|---|---|---|---|---|
| 1 | Present Invention (undetachable) | 32.09 | 113.7 | 3.50 | 1.35 √ |
| 2 | Present Invention (undetachable) | 32.68 | 131.8 | 3.96 | 1.91 |
| 3 | Jockey | 32.42 | 46.1 | 1.37 | 1.53 |
| 4 | Always Discreet | 53.39 | 216.8 | 3.27 | 42.41 |
| 5 | KC Silhoutte | 63.98 | 111.2 | 1.65 | 5.70 |

thereby proving the maximum bodily fluid protection amongst the panties tested. From the foregoing, it will be appreciated that a protective garment according to the present invention provides improved bodily fluid leakage protection, while providing comfortable and attractive look and feel for the protective garment.

In general, the foregoing description is provided for exemplary and illustrative purposes; the present invention is not necessarily limited thereto. Rather, those skilled in the art will appreciate that additional modifications, as well as adaptations for particular circumstances, will fall within the scope of the invention as herein shown and described and of the claims appended hereto.

## Claims

1. A garment for preventing bodily fluid leakage and sagging of an absorbent pad, the garment comprising:
an undergarment; and
a sling placed adjacent to a crotch portion of the undergarment, the sling designed and configured to provide a longitudinal stretch to move with a body of a wearer and to exert a garment force against the absorbent pad and the body; and
wherein the sling is removable.

2. The garment of claim 1, further comprising an absorbent pad shaped and sized to fit within the sling, the absorbent pad designed and configured to exert a pad force.

3. The garment of claim 2, wherein a combination of the garment force and pad force allows bodily fluid containment and low bodily fluid leakage

4. The garment of claim 1, wherein system efficiency is greater than 3.2 and the leakage rate is less than 2.0.

5. The garment of claim 3, wherein the combination of the garment force and pad force promotes longitudinal fluid transport and minimizes radial distribution of bodily fluid.

6. The garment of claim 1, wherein the sling is made out of a material which includes a substrate, a polyurethane material, and an adhesive.

7. The garment of claim 6, wherein the substrate is relatively inelastic.

8. The garment of claim 7, wherein the adhesive is a hot melt adhesive having a melt temperature range of 260 °F (399,8 °K) to 350 °F (449, 8 °K)

9. The garment of claim 1 wherein the sling has a shape of an hourglass.

10. The garment of claim 9 wherein the sling is made with a composite stretchable fabric having at least) two outer layers of nonwoven fabric and an inner layer of elastomeric fibers and between ten and 35 percent by weight of the composite fabric of an adhesive composition bonding potions of the outer and inner layers.

11. The garment of claim 10 wherein the outer layers of nonwoven fabric comprise spandex or elastomer strands to provide both a fit function and fluid absorption function.

## Patentansprüche

1. Bekleidungsstück zum Verhindern des Austretens von Körperfluid und Durchhängen eines absorbierenden Kissens, wobei das Kleidungsstück umfasst:
ein Unterbekleidungsstück; und
eine Schlinge, die benachbart zu einem Schrittteil des Unterbekleidungsstücks angeordnet ist, wobei die Schlinge dafür ausgelegt und gestaltet ist, eine Längsdehnung bei Bewegung des Körpers eines Trägers zu bieten und eine Kleidungskraft gegen das absorbierende Kissen und den Körper auszuüben; und
wobei die Schlinge entfernbar ist.

2. Kleidungsstück gemäß Anspruch 1, ferner umfassend ein absorbierendes Kissen, das geformt und bemessen ist, in die Schlinge zu passen, wobei das absorbierende Kissen dafür ausgelegt und gestaltet ist, eine Kissenkraft auszuüben.

3. Kleidungsstück gemäß Anspruch 2, wobei eine Kombination der Kleidungskraft und der Kissenkraft das Zurückhalten von Körperfluid und geringes Austreten von Körperfluid ermöglicht.

4. Kleidungsstück gemäß Anspruch 1, wobei die Systemwirksamkeit höher als 3,2 und die Austrittsrate kleiner als 2,0 ist.

5. Kleidungsstück gemäß Anspruch 3, wobei die Kombination der Kleidungskraft und der Kissenkraft längsgerichteten Fluidtransport fördert und radiale Verteilung von Körperfluid minimiert.

6. Kleidungsstück gemäß Anspruch 1, wobei die Schlinge aus einem Material besteht, das ein Substrat, ein Polyurethanmaterial und einen Klebstoff enthält.

7. Kleidungsstück gemäß Anspruch 6, wobei das Substrat vergleichsweise unelastisch ist.

8. Kleidungsstück gemäß Anspruch 7, wobei der Klebstoff ein Heißschmelzklebstoff mit einem Schmelztemperaturbereich von 260 °F (399,8 °K) bis 350 °F (449,8 °K) ist.

9. Kleidungsstück gemäß Anspruch 1, wobei die Schlinge die Form einer Sanduhr aufweist.

10. Kleidungsstück gemäß Anspruch 9, wobei die Schlinge mit einem dehnbaren Verbundgewebe aus wenigstens zwei äußeren Schichten von Vliesgewebe, einer inneren Schicht von Elastomerfasern und zwischen zehn und 35 Gewichtsprozent, bezogen auf das Verbundgewebe, einer Klebstoffzusammensetzung, die Teile der äußeren und inneren Schichten verbindet, gefertigt ist.

11. Kleidungsstück gemäß Anspruch 10, wobei die äußeren Schichten von Vliesgewebe Spandex- oder Elastomerstränge umfassen, um sowohl eine Passfunktion als auch eine Fluidabsorptionsfunktion bereitzustellen.

## Revendications

1. Vêtement permettant d'éviter une fuite de liquide corporel et l'affaissement d'un tampon absorbant, le vêtement comprenant :
un sous-vêtement ; et
une bandelette placée de manière adjacente à une partie entrejambe du sous-vêtement, la bandelette étant conçue et configurée pour permettre d'obtenir un étirement longitudinal pour se déplacer avec le corps d'un utilisateur et pour exercer une force de vêtement à l'encontre du tampon absorbant et du corps ; et
dans lequel la bandelette est amovible.

2. Vêtement selon la revendication 1, comprenant en outre un tampon absorbant formé et dimensionné pour s'ajuster à l'intérieur de la bandelette, le tampon absorbant étant conçu et configuré pour exercer une force de tampon.

3. Vêtement selon la revendication 2, dans lequel une association de la force de vêtement et de la force de tampon permet un confinement du liquide corporel et une faible fuite du liquide corporel.

4. Vêtement selon la revendication 1, dans lequel l'efficacité du système est supérieure à 3,2 et le taux de fuite est inférieur à 2,0.

5. Vêtement selon la revendication 3, dans lequel l'association de la force de vêtement et de la force de tampon favorise un transport longitudinal du liquide et réduit au minimum la répartition radiale du liquide corporel.

6. Vêtement selon la revendication 1, dans lequel la bandelette est faite d'un matériau qui comprend un substrat, un matériau en polyuréthane et un adhésif.

7. Vêtement selon la revendication 6, dans lequel le substrat est relativement inélastique.

8. Vêtement selon la revendication 7, dans lequel l'adhésif est un adhésif thermofondu ayant une plage de température de fusion de 260 °F (399,8 °K) à 350 °F (449,8 °K).

9. Vêtement selon la revendication 1 dans lequel la bandelette a la forme d'un sablier.

10. Vêtement selon la revendication 9 dans lequel la bandelette est faite d'un tissu étirable composite comportant au moins deux couches externes de tissu non tissé et une couche interne de fibres élastomères et entre dix et 35 pour cent en poids du tissu composite d'une composition adhésive liant des parties des couches externes et interne.

11. Vêtement selon la revendication 10 dans lequel les couches externes de tissu non tissé comprennent des brins de spandex ou d'élastomère pour fournir à la fois une fonction d'ajustement et une fonction d'absorption du liquide.
